# EUROPEAN PATENT APPLICATION

(11) **EP 0 958 819 A1**
(43) Date of publication of application: **24.11.1999**
(21) Application number: 97912444.3
(22) Date of filing: 13.11.1997
(51) Int. Cl.: A61K 31/435

(54) **REMEDIES/PREVENTIVES FOR ABNORMAL MOTIONS ACCOMPANYING DISORDERS IN EXTRAPYRAMIDAL SYSTEM**

(30) Priority: 18.12.1996 JP 33825196
(71) Applicant: Asahi Kasei Kogyo Kabushiki Kaisha, Osaka-shi, Osaka 530-8205 (JP)
(72) Inventor: MOCHIZUKI, Daisuke, Tagata-gun, Shizuoka 410-21 (JP); HOKONOHARA, Tadami, Tagata-gun, Shizuoka 410-23 (JP)
(74) Representative: BROOKES & MARTIN
(86) International application number: JP9704135
(87) International publication number: WO9826778

(57) **Abstract**

Preventives/remedies for abnormal motions accompanying disorders in the extrapyramidal system which contain as the active ingredient (1S)-3-[2-(2-adamantyl)ethyl]-1,8,8-trimethyl-3-azabicyclo[3.2.1]-octane or a pharmacologically acceptable acid-addition salt thereof. These drugs are novel and efficacious preventives/remedies for a disease selected from the group consisting of Parkinson's disease, striatonigral degeneration, olivopontocerebellar atrophy, Shy-Drager syndrome, progressive supranuclear palsy, essential tremor, Huntington's chorea, senile chorea, fastigiobulbar pallidial Luys body atrophy, benign familial chorea, acanthocytic chorea, torsion dystonia, Meige's syndrome, and Segawa disease.

## Description

### Field of Industrial Application

This invention relates to a preventives/remedies for abnormal movements, such as hypokinesia, chorea and dystonia, accompanying disorders in the extrapyramidal system which contain as the active ingredient (1S)-3-[2-(2-adamantyl)ethyl]-1,8,8-trimethyl-3-azabicyclo [3.2.1]-octane or a pharmacologically acceptable acid-addition salt thereof.

### Prior arts

Parkinson's disease is a disease reported at first by British physician, James Parkinson, in 1817. Its incidence is 100 - 180/100,000 persons in Europe and in the U.S., mostly crisis in 50 years old ages. In the Parkinsonism, abnormal movements such as specified by tremor, muscular rigidity and hypokinesia are observed. Parkinsonism is a degenerative disease in the brain, in which the deciduous degeneration of dopaminergic cells in substantia nigra of a part of the extrapyramidal system is observed. It is well known that in case of extrapyramidal system is injured, as a result, abnormal movements occurs (Kazuro Sugiura, "Understanding of the central nerves system by illustration", page 74, Ishiyaku Publ. Co., Hideo Togi, Clinical Neuroscience 13, 394, 1995).

The extrapyramidal system controls motor systems with unconscious cooperation in order to take smooth volitional movement. For example, it is roles of the extrapyramidal system that when one intends to bent arm, the flexors are contracted by his own volition, simultaneously muscle relaxation of the extensor of opposite position occurs unconsciously or preferable regulatory force will be added for preventing excess or insufficient movements. Consequently, if the extrapyramidal system is damaged, smooth movement can not be possible, or involuntary movements, i. e. abnormal movements, will occur.

The damage in extrapyramidal system is known to occur with not only Parkinson's disease but the various factors, degenerative disease such as Huntington's chorea, metabolic disorder such as Wilson's disease, inflammatory disease such as encephalitis, cerebrovascular disorders such as cerebral infarction and adverse drug reactions and so on.

Abnormal movements accompanied with disorder of extrapyramidal system can mainly be classified into following three major symptoms (Nozomu Suwa, "Modern Psychosomatic Medicine", p. 111, Nankodo Publ.).
① A group with major symptom of tremor, muscle rigidity or hypokinesia;
② A group with major symptom of rapid involuntary movement and muscle hypotonia
③ A group with major symptom of slow involuntary movement and hypermyotonia.

Surgical treatment with stereotaxic operation is performed for treatment of abnormal movement caused by the disorder of extrapyramidal system, but this is not commonly used, and pharmacotherapy is applied mainly. The pharmacotherapy differs according to the above classification.

### ① A group with major symptom of tremor, muscle rigidity or hypokinesia

In case of major symptom of tremor, muscle rigidity or hypokinesia, For treatment of this group, anticholinergic drugs, L-dopa or dopamine receptor agonists are used. However, as a result of these drug administration, adverse drug reaction as like abnormal movement in the group ② will offen appear.

### ② A group with major symptom of rapid involuntary movement and muscle hypotonia

For treatment of this group, mainly dopamine receptor blocking agents are used. The high incidence of adverse drug reactions caused by the drug, such as tremor, muscle rigidity, hypokinesia, dyskinesia, akathisia and dystonia, will appear. No drug is known for treatment of delayed dyskinesia, which occurs due to long term administration of dopamine receptor blocking agent.

### ③ A group with major symptom of slow involuntary movement and hypermyotonia

Anticholinergic drug, dopamine receptor blocker, dopamine receptor agonist, antidepressant, anxiolytics or anticonvulsants is tried for treatment, but no constant evaluation is obtained.

Symptoms of these three groups not only occur independently but also appear with multiple types of symptoms. Consequently, treatment for abnormal movements accompanied with the extrapyramidal system disorder is very complex and difficult, and actually it is the present condition that treatments with trial and error are conducted for individual symptom.

### Problem to be solved by the invention

This invention relates to novel and useful preventives or remedies for abnormal movements accompanying disorders in the extrapyramidal system.

### Means for solving problem

The present inventors have studied to solve the above problem and found that, unexpectedly, (1S)-3-[2-(2-adamantyl)ethyl]-1,8,8-trimethyl-3-azabicyclo [3.2.1]-octane had excellent efficacy for treatment of abnormal movements accompanied with extrapyramidal disease, and completed the present invention.

An object of the present invention is to provide preventives or remedies (hereinafter sometimes designates as preventives/remedies) for abnormal movements accompanying disorders in the extrapyramidal system which contain as the active ingredient (1S)-3-[2-(2-adamantyl)ethyl]-1,8,8-trimethyl-3-azabicyclo [3.2.1]-octane or a pharmacologically acceptable acid-addition salt thereof.

Another object of the present invention is to provide preventives or remedies for abnormal movements accompanying disorders in the extrapyramidal system, in which the said abnormal movements are more than any one of abnormal movement with major symptom of tremor, muscle rigidity or hypokinesia, abnormal movement with major symptom of rapid involuntary movement and muscle hypotonia or abnormal movement with major symptom of slow involuntary movement and hypermyotonia.

More another object of the present invention is to provide preventives or remedies for abnormal movements accompanying disorders in the extrapyra-midalsystem, in which the said abnormal movements are selected from the group consisting of tremor, muscle rigidity, hypokinesia, chorea, ballismus, dyskinesia, akathisia, dystonia and athetosis.

Further object of the present invention is to provide preventives or remedies for abnormal movements accompanying disorders in the extrapyramidal system, in which the said abnormal movements are side effect of drug abnormal movements.

More further object of the present invention is to provide preventives or remedies containing (1S)-3-[2-(2-adamantyl)ethyl]-1,8,8-trimethyl-3-azabicyclo [3.2.1]-octane or a pharmacologically acceptable acid-addition salt thereof asthe active ingredient, for a disease selected from the group consisting of Parkinson's disease, striate-nigral degeneration, olivoponto-cerebellar atrophy, Shy-Drager syndrome, progressive supranuclear palsy, essential tremor, Huntington's chorea, senile chorea, dentate-rubro-pallido-luysian atrophy, benign familial chorea, chorea-acanthocytosis, dystonia musculorum deformans, Meige's syndrome and Segawa disease.

The extrapyramidal system is a motor system reflectively innervating skeletal muscle, excluding the motor neurons of pyramidal system innervating voluntary movement. Namely, the extrapyramidal system regulates unconscious motions of sketal muscle such as tone of muscle and coordination of muscular motions, and has a role to realize ordinary movement and maintain somatic equilibrium. Consequently, disorders of extrapyramidal system cause to non-smooth motion, involuntary motion and impossible voluntary regulation, in other words, abnormal movements. Namely, abnormal movements accompanied with disorders of extrapyramidal system is a general term of motor disorders occurred due to impossible to keep normal motion and to maintain tone of skeletal muscle caused by disorders of extrapyramidal system.

Such the abnormal movement means involuntary and non-regular motions, which can not occur in the normal subject. Consequently, though the reflex movement occurs without voluntary sense, it is a regular movement in the normal subject, hence it is not included in the abnormal movement. Abnormal movements are also designated as movement disorders, hence this is interpreted as synonym. Similar term involuntary movements indicate as same movement of involuntary, non-regular and not generated in the normal subjects, but these do not include muscle rigidity and hypokinesia.

Anatomical definition of a term of extrapyramidal system is somewhat different in the specialists and is understood to indicate regions including cerebral cortex, thalamus, putamen, caudate nucleus, pallidum, subthalamic nucleus, red nucleus, substantia nigra, reticular nuclei of the brain stem, nucleus olivaris, vestibular nucleus and cerebellum. Further, putamen and caudate nucleus are totally called as corpus striatum and putamen and pallidum are totally called as nucleus lentiformis. Also, putamen, caudate nucleus, pallidum, subthalamic nucleus, red nucleus and substantia nigra are totally called as lenticular nucleus. Also, putamen, caudate nucleus, pallidum, subthalamic nucleus, red nucleus and substantia nigra are totally as basal nuclei.

As described hereinbelow, disorders of extrapyramidal system occurs due to various causes, for example degenerative disease such as Parkinson's disease and Huntington's chorea, metabolic diseases such as Wilson's disease and hyperthyroidism, inflammatory diseases such as encephalitis and systemic lupus erythematosus, vascular failures such as cerebral infarction and cerebral hemorrhage, side effect of drug, tumors and toxic diseases caused by such as mercury and manganese, and so on. Consequently, it may be more substantial to classify by specific features of abnormal movements than the causes of it.

The abnormal movements accompanied with disorders of the extrapyramidal system can be classified into the following three major symptoms by degree of speed of abnormal movements or state of tonus. Further, these can be classified in details by specificity of the abnormal movements. Following are classification of the abnormal movements and characteristics mainly of macroscopic observation.

### ① A group with major symptom of tremor, muscle rigidity or hypokinesia

(A) Tremor: rapid, regular and repetitive abnormal movements; sometimes tremor occurs in fatigue or stress, but is called as physiological tremor and is not abnormal movement. Abnormal tremor includes obvious oscillation of hands, arms or legs in resting state (resting tremor), occurring tremor during the performance (action tremor), occurring tremor being kept in certain position (postural tremor) and occurring tremor to approch intentionally finger tip to the target (intention tremor), and is included in the abnormal movement.
(B) Muscular rigidity: rigid state with occurring continuous resistance against periarticular passive motion; strong resistance will occur when bending or extending joint due to hypermyotonia of flexors and extensors.
(C) Hypokinesia: diminished or lost coordination and difficult to initiate movement; delay of initiation of movement for walking with impossible of leg to move forward in spite of having intention. Synonym: akinesia and bradykinesia.

### ② A group with major symptom of rapid involuntary movement and muscle hypotonia

(A) Chorea: irregular, spasmodic, involuntary movement of the limbs, facial and somatic muscles.
(B) Ballismus: more rapid and violent abnormal movement than chorea; manifested as jerking, flinging movements of the extremity; relatively regular movements and repeated involuntary movements three times in a second; usually only one side of the body is involved.
(C) Dyskinesia: irregular abnormal movements slower than chorea occurring localized in lingual, labial, facial and buccal region; motions of lingua with twisting or thrusting and oral movement as like eating food. Also called as orobuccolingual dyskinesia.
(D) Akathisia: abnormal movement characterized by inability of maintaining lower limbs at resting position, always with movements of standing and sitting posture and stepping. Usually accompanying with sensory abnormality of itching feeling and anxiety and stress feeling.

### ③ A group with major symptom of slow involuntary movement and hypermyotonia

(A) Dystonia: slow involuntary movement occurring irregular posture (dystonic posture) or involuntary movements due to muscular contraction of agonist and excess contraction of antagonist at the maintaining posture and voluntary motions; Usually accompanying with pain; and occurring systemic or localized with cervix or end of limbs.
(B) Athetosis: involuntary movements with irregular, rough, continuous, more rapid than movements of dystonia but slow movement of end of limbs or facial surface

Diagnosis of abnormal movement is performed at first by macroscopic observation. Further, since the abnormal movement differs in the condition of resting or moving and time dependently changing, video imaging of the symptom for objective diagnosis is used. In this diagnosis, differentiation is not always easy. Consequently, analyzing surface electromyogram and searching the cause of abnormal movements are performed for total diagnosis.

Factors causing aforesaid three major symptoms are very wide diversity. Following are illustrative of major causes of crisis.

### ① A group with major symptom of tremor, muscle rigidity or hypokinesia

(a) side effect of drug for example adverse drug reaction caused by the following drugs
   Dopamine receptor blockers such as chlorpromazine, triflupromazine, levomepromazine, thioridazine, propericyazine, perazine, prochiorperazine, trifluoperazine, thioproperazine, fluphenazine, perphenazine, haloperidol, spiperone, bromperidol, pipamperone, moperone, timiperone, tiotixene, chlorprothixene, flupentixol, sulpiride, sultopride, nemonapride, tiapride, clotiapine, zotepine, pimozide, carpipramine, clocapramine, mosapramine, oxypertine, clozapine, loxapine, olanzapine, risperidone, domperidone and metoclopramide, monoamine depletors such as reserpine and dopamine precursors such as L-dopa.
(b) degenerative diseases: Parkinson's disease, striate-nigral degeneration, olivepontocerebellar atrophy, Shy-Drager syndrome, progressive supranuclear palsy and essential tremor and so on.
(c) metabolic diseases: Wilson's disease, hyperthyroidism and type III G_{M1}-gangliosidosis and so on.
(d) inflammatory diseases: encephalitis and so on.
(e) angiopathic diseases: cerebral infarction, cerebral hemorrhage and head injury and so on.
(f) toxic diseases: MPTP poisoning, manganese poisoning and carbon monoxide poisoning and so on.
(g) others: cerebral palsy, normal pressure hydrocephalus and brain tumor and so on.

### ② A group with major symptom of rapid involuntary movement and muscle hypotonia

(a) side effect of drug : adverse drug reactions caused by drugs described in the above ①-(a). Except for the above described drugs, dopaminergic agonists such as bromocriptine and apomorphine. Anticholinergic agents such as trihexyphenidyl, biperiden and ethopropazine. Drugs for treatment of manic-depressive psychosis such as lithium. Oral contraceptives.
(b) Degenerative diseases: Huntington's disease, hemiballismus, senile chorea, dentate-rubro-pallido-luysian atrophy, benign familial chorea and chorea-acanthocytosis and so on.
(c) Metabolic diseases: Wilson's disease, hyperthyroidism, Lesch-Nyhan syndrome and phenylketonurias and so on.
(d) Inflammatory diseases: encephalitis, systemic lupus erythernatosus, typhoid and diphtheria and so on.
(e) angiopathic diseases: cerebral infarction, cerebral hemorrhage and head injury and so on.
(f) toxic diseases: mercury poisoning, lithium poisoning and carbon monoxide poisoning and so on.
(g) others: cerebral palsy and brain tumor and so on.

### ③ A group with major symptom of slow involuntary movement and hypermyotonia

(a) side effect of drug : adverse drug reactions caused by drugs described in the above ① (a).
(b) degenerative diseases: dystonia musculorum deformans, Huntington's disease, striate-nigral degeneration, progressive supranuclear palsy, Meige's syndrome, Joseph's disease and Hallervorden-Spatz disease and so on.
(c) metabolic diseases: Wilson's disease, Lesch-Nyhan syndrome, G_{M1}-gangliosidosis and G_{M2}-gangliosidosis and so on.
(d) inflammatory diseases: encephalitis and so on.
(e) angiopathic diseases: cerebral infarction, cerebral hemorrhage and head injury and so on.
(f) toxic diseases: manganese poisoning, hydrogen sulfide poisoning and carbon monoxide poisoning and so on.
(g) others: cerebral palsy and brain tumor and so on.

As explained hereinabove, side effect of drug abnormal movement has been well known, and in clinical point of view, extrapyramidal disorder caused by drugs having dopamine receptor blocker is specifically important problem. These abnormal movements include muscle rigidity, hypokinesia, dyskinesia, akathisia and dystonia. A positive correlation is observed between therapeutic effect of dopamine receptor blocking agents on schizophrenia and occurrence of the abnormal movements, consequently maintaining the drug efficacy as well as reducing the abnormal movements are very difficult. Since within several hours to one month after administration of antischizophrenic drug, acute abnormal movements occur prevalently, combined administration of anticholinergic agent is initiated simultaneously at the initial time of administration. Adverse drug reaction of anticholinergic agent occurs, however, for example, thirsty, constipation and ischuria. Furthermore, long term administration over one year of antischizophrenic drug results to prevalently occur delayed dyskinesia, delayed dystonia and delayed akathisia. For these delayed abnormal movements, the fact that not only to have no remedy for effective treatment but also to deteriorate the delayed abnormal movements by administration of anticholinergic agent is known. Consequently, the abnormal movements occurring by administration of antischizophrenic drug are very much obstacle to treatment of schizophrenia. Induction of dyskinesia as an adverse drug reaction of L-dopa, a drug for treatment of Parkinson's disease, which is specified by tremor, muscle rigidity and hypokinesia, is known.

As explained hereinabove, the side effect of drug abnormal movement, which is one of the abnormal movements accompanied with disorders of extrapyramidal system, including ① a group with major symptom of tremor, muscle rigidity or hypokinesia; ② a group with major symptom of rapid involuntary movement and muscle hypotonia; and ③ a group with major symptom of slow involuntary movement and hypermyotonia is clinically observed in general, but is difficult to treat. The present invention provides superior remedies/preventives for side effect of drug abnormal movements.

Various degenerative diseases have been shown hereinabove as causes of abnormal movemenrs including ① a group with major symptom of tremor, muscle rigidity or hypokinesia; ② a group with major symptom of rapid involuntary movement and muscle hypotonia; and ③ a group with major symptom of slow involuntary movement and hypermyotonia, however, on the contrary, these diseases may be thought as the diseases at first when abnormal movements of these group of ①, ② and ③ occur. Consequently, the present invention can be provided as the other point of view including remedies/preventives for diseases showing the above abnormal movements.

Namely, (1S)-3-[2-(2-adamantyl) ethyl]-1,8,8-trimethyl-3-azabicyclo [3.2.1]-octane or a pharmacologically acceptable acid-addition salt thereof of the present invention relates to remedies/preventives for a disease selected from the group consisting of Parkinson's disease, striate-nigral degeneration, olivopontocerebellar atrophy, Shy-Drager syndrome, progressive supranuclear palsy, essential tremor, Huntington's chorea, senile chorea, dentate-rubro-pallido-luysian atrophy, benign familial chorea, chorea-acanthocytosis, torsion dystonia, Meige's syndrome and Segawa disease.

In addition, the clinical features and differential diagnoses of these diseases are described hereinbelow.
(1) Parkinson's disease: A syndrome characterized by tremor, muscle rigidity, akinesia (bradykinesia) and disorder of postural reflex. Tremor is observed at rest (resting tremor), and in the other characteristics of muscle rigidity, akinesia (bradykinesia) and disorder of postural reflex, any one of crisis is observed, it may be highly possible onset of Parkinsonism. Parkinsonism induced by drug can be differentiated by administration of causative drug or not, and angiopathic crisis can be differentiated by CT scanning.
(2) Striate-nigral degeneration: It is similar to Parkinson's disease, but make more rapid worse than Parkinson's disease. Moreover, L-dopa, an anti-parkinsonism drug, is ineffective. Finally, this disease can be differentiated by MRI (magnetic resonance imaging).
(3) Olivopontocerebellar atrophy: Prevalently onset is in ages between 20 and 60 years old. In the initial stage, gait disorder will occur. As the disease progresses, it combines with nystagmus, aphasia, muscle rigidity and hypokinesia. Diagnosis can be made by MRI with significant atrophy of pons.
(4) Shy-Drager syndrome: The disease characterized by dizziness with orthostatic hypotension, syncope, muscle rigidity, hypokinesia and gait disorder. It can be differentiate with orthostatic hypotension from Parkinson's disease.
(5) Progressive supranuclear palsy: Onset in the elderly with death within 3 - 7 years. It is characterized by dystonia of cervix or the upper half of the body, disturbance of ocular motility, muscle rigidity and hypokinesia. Tremor is postural tremor and can be differentiated from resting tremor in Parkinson's disease.
(6) Essential tremor: Major symptom with postural tremor, disappeared at rest. No disorder occurs other than the tremor. No progress is seen.
(7) Huntington's chorea: Hereditary disease of key finding with dancing movement (chorea) and dementia. Diagnosis is determined by familial dominant inheritance and atrophy of caudate nucleus.
(8) Senile chorea: Dancing movement of elderly. The symptom is not hereditary disease and no dementia is observed.
(9) Dentate-rubro-pallido-luysian atrophy: Disease of onset prevalently onset from 15 to 40 years old, characterized by chorea gait disorder and nystagmus. Definite diagnosis is performed by CT scan with atrophies of cerebellar dentate nucleus, red nucleus, pallidal outer segment and Luys body.
(10) Benign familial chorea: Hereditary disease, onset in juvenile age. Chief symptom is chorea and intelligence us normal.
(11) Chorea-acanthocytosis: Hereditary disease, which onset young generation from 10 to 20 years old generations, characterized by chorea, autophagia and increased acanthocytes. Autophagia means a symptom to bite obsessively own lips, etc. Its chorea can not differentiate from Huntington's chorea. Differentiation can be made from symptoms showing autophagia and increased acanthocytes in blood examination.
(12) Dystonia musculorum deformans: Hereditary disease with key finding of dystonia. Prevalently onset on 5 - 15 years old. Dystonia occurs in the limbs, cervix and body. Decreased level of β-galactosidase can be useful for differentiation from other diseases accompanied with dystonia.
(13) Meige's syndrome: Chief symptoms are facial dystonia and blepharospasm.
(14) Segawa's disease: Mainly onset in females, 4 - 10 years old, characterized by dystonia posture of limbs induced by gait. Its characteristics are dystonia posture with no dystonia movements, and can be differentiated from dystonia musculorum deformans with dystonia movements.

Animal models of abnormal movements accompanied with disorders of extrapyramidal system are known in the following.

### ① Animal model for a group with major symptom of tremor, muscle rigidity or hypokinesia

Catalepsy model: When dopamine receptor blocker is administered in rats, enforced posture is maintained. (Munkvad, I et al. Brain Behav. Evol., 1, 89 - 100, 1968)

### ② Animal model of a group with major symptom of rapid involuntary movement and muscle hypotonia

IDPN model: When 3,3-iminodipropionitrile (IDPN) is administered in rats, abnormal movements characterized by rapid involuntary movement and muscle hypotonia appear, (Schneider, J.S., Exp. Neurol., 84, 524 - 532, 1984)

### ③ Animal model of a group with major symptom of slow involuntary movement and hypermyotonia

Dystonia model: An unilateral microinjection of haloperidol into the red nucleus of rat produces torsional movements of head, that is dystonia, (Matsumoto, R.R. et al., Pharmacol. Biochem. Behavior.,36, 151 - 155, 1990)

### ④ Animal model for electrophysiological measurement of disorders of extrapyramidal system

Red nuclei belong the extrapyramidal system and regulates motor functions. Relationship between hypofunction of red nuclei and abnormal movements is reported (Kolasa. K. et al. Pharmacol. Biochem. Behav. 51, 29 - 35, 1995), consequently measurement of function of red nuclei will be important. Function of red nuclei can be determined by measuring firing rate electrophysiologically.

Preventives/remedies of the present invention is included as an active ingredient (1S)-3-[2-(2-adamantyl) ethyl]-1,8,8-trimethyl-3-azabicyclo [3.2.1]-octane(hereinafter designates as compound A) or a pharmacologically acceptable acid-addition salt thereof. The compound A or a pharmacologically acceptable acid-addition salt thereof is disclosed, for example, in Japanese Patent Unexamined Publication No. Hei 8-109129 as showing antischizophrenic activity and its manufacturing process is also disclosed.

The compound A of the present invention can optionally be converted as a pharmacologically acceptable acid-addition salt thereof. Examples of these salts are salt with inorganic acid such as hydrochloric acid, sulfuric acid and phosphoric acid, and acid addition salt with organic acid such as acetic acid, propionic acid, tartaric acid, citric acid, glycolic acid, gluconic acid, succinic acid, malic acid, glutamic acid, aspartic acid, methanesulfonic acid, mandelic acid, p-toluenesulfonic acid and maleic acid.

No death was observed when the compound A of the present invention or acid addition salt thereof is administered orally in mice at 1 g/kg, which shows safety for use as pharmaceutical product. Preventives/remedies of the present invention was confirmed their effect on abnormal movement models accompanied with disorders of extrapyramidal system as well as showing low toxicity. Consequently, preventives/remedies of the present invention are confirmed as the excellent preventives/remedies for abnormal movements accompanied with disorders of extrapyramidal system.

The compound A of the present invention or acid addition salt thereof can be used as pharmaceutical preparation by formulation thereof, usually for oral administration or parenteral administration such as injection including drip infusion. Its dose amount is depending on form of administration and age, body weight and symptom of patient, and is usually 0.01 mg - 100 mg /man/day.

Examples of formulations of the above preparation are tablets, pills, powders, granules, capsules and injections. For manufacture of these preparations, various additives can be used. These are, for example for use in oral use such as tablets, granules and capsules, additives such as starch, lactose, sucrose, mannitol, carboxymethylcellulose, corn starch and inorganic salts, binders such as starch, dextrin, acacia, gelatin, hydroxypropyl starch, methylcellulose, carboxymethylcellulose sodium, hydroxypropylcellulose, crystalline cellulose, ethylcellulose, polyvinyl pyrrolidone and macrogol, disintegrators such as starch, hydroxypropyl starch, carboxypropylcellulose, carboxymethylcellulose sodium and hydroxypropylcellulose, surface active agents such as sodium lauryl sulfate, soybean lecithin, sucrose esters of fatty acidsand polysorbate 80, lubricants such as talc, wax, hydrogenated plants oil, sucrose esters of fatty acids, magnesium stearate and magnesium stearate, fluidity promoter, correctives, coloring agents and perfumes.

The compound A of the present invention or acid addition salt thereof can be used as suspension, emulsion, syrup and elixirs. In parenteral preparation, water for injection, physiological saline, glucose aqueous solution, vegetableoil for injection, propylene glycol and polyethylene glycol can be used for dilution. Further, if necessary, bactericides, antiseptics, antioxidants, stabilizing agents, tonicity agents and soothing agents can optionally be added.

Following examples illustrate the present invention but are not construed as limiting.

### Examples

In order to indicate effect of the preventives/remedies of the present invention, following experiments were conducted using the compound A hydrochloride of the present invention as a drug for testing. The compound A hydrochloride was prepared according to the description of example 15 in Japanese Patent Unexamined Publication No. Hei 8-109129.

### Example 1

### Preventive effect for catalepsy model

When a drug having dopamine receptor blocking activity is administered in rats, catalepsy induced. Catalepsy means a symptom of rats, which can not recovered by own intention, when rats are enforced unnatural posture, and it reflects abnormal movements consisting of mainly tremor, muscle rigidity and hypokinesia. (Hornykiewicz, O. Br. Med. Bull., 29, 172 - 178, 1973). Namely, if pretreatment of drug shows antagonistic action against catalepsy, the said drug is decided have a preventive effect on abnormal movements accompanied with disorders of extrapyramidal system mainly consisting of tremor, muscle rigidity and hypokinesia of humans. (Schmidt, W.J. and Bubser, M., Pharmacol. Biochem. Behavior., 32, 621 - 623, 1988).

### 1. Method

The compound A hydrochloride, 1mg/kg, was administered orally into Wistar rats, male (Japan Charles River Inc.). After 30 minutes, haloperidol (Sigma Corp., U.S.A.), 2mg/kg, or risperidone, which was extracted from Risperdal (trade name, Janssen Corp.,UK) according to the method hereinbelow explained, 20 mg/kg were administered intraperitoneally. Catalepsy after 1 hour of administration of haloperidol or risperidone was observed. Catalepsy was observed by hanging rats in their forepaws on the iron bar, diameter 2 mm, set on the position of 12 cm high, and we measured the time maintaining this posture. Measurements were conducted maximum for 180 seconds. Experiments were conducted in a group of 10 rats for haloperidol induced catalepsy test and a group of 5 rats for risperidone induced catalepsy test. The mean duration of catalepsy in distilled water (vehicle of the compound in present invention)-administered group was defined as 100 %. Suppressive rate was indicated as percent of the shortening on the duration of catalepsy in the compound-administered group comparing with the mean of that in distilled water administered group.

Risperidone used in examples in the present invention was prepared as follows. Risperdal Tab. (Janssen Corp. U.K., 4 mg Tab., 900 tablets) was crushed in the mortar and transferred into flask. Chloroform 750 ml was added thereto and extracted with stirring at room temperature for 90 minutes. Insoluble was suctioning filtered and the filtrate was concentrated in vacuo. The thus obtained residue was purified by silica gel column chromatography (Wako gel. C200. chloroform: methanol = 15 : 1 - 10 : 1) to obtain risperidone 3.5 g, purity over 99.5 %.

### 2. Results

Test drug used: haloperidol. Preventive effect of the compound A on haloperidol-induced catalepsy is shown in Table 1 hereinbelow.

**Table 1**

| Drug | Suppressive rate (mg/kg, p.o.) |
|---|---|
| Compound A hydrochloride | 77% (1) |

Test drug used: risperidone. Preventive effect of the compound A on risperidone-induced catalepsy is shown in Table 2 hereinbelow.

**Table 2**

| Drug | Suppressive rate (mg/kg, p.o.) |
|---|---|
| Compound A hydrochloride | 53% (1) |

As a result, the compound used in the present invention has strong preventive effects against catalepsy induced by haloperidol and risperidone, which are remedies for schizophrenia and have dopamine receptor blocking action. Consequently, the compound used in the present invention is effective for prevention of abnormal movements with main symptoms of tremor, muscle rigidity and hypokinesia accompanied with disorders of extrapyramidal system.

### Example 2

### Therapeutic effect against catalepsy model

### 1. Method

Therapeutic effects of the compound of the present invention on haloperidol or risperidone induced catalepsy were examined using Wistar rats, male (Charles River Inc., Canada).

In haloperidol administered group, rats, to which haloperidol 2 mg/kg was administered intraperitoneally and showed catalepsy after 30 minutes, were used. After further 15 minutes, the compound of the present invention or distilled water was administered orally. Therapeutic effect of the present compound against haloperidol induced catalepsy was measured after 75 minutes of administration of the compound of the present invention.

In risperidone administered group, rats, to which risperidone 20 mg/kg was administered intraperitoneally and showed catalepsy after 60 minutes, were used. After further 15 minutes, the compound of the present invention or distilled water was administered orally. Therapeutic effect of the present compound against risperidone induced catalepsy was measured after 105 minutes of administration of the compound of the present invention.

Catalepsy was observed by hanging rats in their forepaws on the iron bar, diameter 2 mm, set on the position of 12 cm high, and we measured the duration of catalepsy. Measurements were conducted maximum for 180 seconds. Experiments were conducted in a group of 10 rats for haloperidol-induced catalepsy test and a group of 5 to 6 rats for risperidone induced catalepsy test. The mean duration of catalepsy in distilled water (vehicle of the compound in present invention)-administered group was defined as 100 %. Suppressive rate was indicated as percent of the shortening on the duration of catalepsy in the compound-administered group comparing with the mean of that in distilled water administered group.

### 2. Results

Therapeutic effect of the compound of the present invention on haloperidol-induced catalepsy is shown in Table 3 hereinbelow.

**Table 3**

| Drug | Suppressive rate (mg/kg, p.o.) |
|---|---|
| Compound A hydrochloride | 50% (1) |

Therapeutic effect of the compound of the present invention on risperidone-induced catalepsy is shown in Table 4 hereinbelow.

**Table 4**

| Drug | Suppressive rate (mg/kg, p.o.) |
|---|---|
| Compound A hydrochloride | 48% (1) |

As a result, the compound used in the present invention has strong preventive effects against catalepsy induced by haloperidol and risperidone, which are remedies for schizophrenia. Consequently, the compound used in the present invention is effective for treatment of abnormal movements with main symptoms of tremor, muscle rigidity and hypokinesia accompanied with disorders of extrapyramidal system.

### Example 3

### Antagonistic action against IDPN model

When 3,3-iminopropyonitrile (IDPN, Tokyo Kasei Kogyo K.K., Japan) is administered in rats, rapid reciprocation of horizontal and vertical head movements is induced. This involuntary movements does not disappear throughout the life if once occurred, is aggravated by stress and is not observed during sleeping. This is very similar to the abnormal movements accompanying disorders of extrapyramidal system of human, which is classified into the group with major symptom of rapid involuntary movement and muscle hypotonia. (Schneider, J. S., Exp. Neurol., 84, 524 - 532, 1984).

### 1. Method

IDPN (Tokyo Kasei Kogyo K.K., Japan) 150 mg/kg was administered to Sprague-Dawley rats (Charles River Inc., 7 weeks old) for 6 days intraperitoneally. Animals expressing horizontal and vertical head movements (at initiation of the experiment: 12 - 14 weeks old) within after 2 weeks of administration were used for dyskinesia model animals. The compound of the present invention (1 mg/kg) or distilled water (as a control group) was administered orally in rats. After 30 minutes, numbers of horizontal and vertical head movements neck during 30 minutes thereafter were counted. One reciprocation is counted as one motion.

### 2. Results

Result is shown in Table 5. (Mean ± standard error in 6 rats was measured.)

**Table 5**

| Drug | Numbers of oscillation of neck |
|---|---|
| Control group | 347 ± 14.4 |
| Compound A administered group | 224.5 ± 24.4** |

| | |
|---|---|
| ** : p < 0.01 | |

The compound of the present invention, 1 mg/kg, administered orally showed inhibitory action against involuntary head-movements induced by IPDN. This result indicated that the compound of the present invention had strong suppressive action against abnormal movements with major symptom of rapid involuntary movement and muscle hypotonia as like chorea, ballismus and dyskinesia.

### Example 4

### Inhibitory effect against dystonia model

Unilateral infusion of haloperidol into red nucleus of rats results to appear continuous torsional movements of the head when the infusion side was laid down under side. This model reflects dystonia characterized by slow involuntary movement and hypermyotonia, which is one of abnormal movements accompanying disorders of extrapyramidal system. (Matsumoto, R.R. et al. Pharmacol. Biochem. Behavior.,36, 151 - 155, 1989). Evaluation of the compound of the present invention on dystonia model was performed by the following method.

### 1. Method

Wistar rats (Japan Charles River Inc., 9 weeks old) were anesthetized by administration of pentobarbital (Somnopentil injection) 50 mg/kg intraperitoneally, and fixed the head by stereotaxic apparatus (David Coop Inc. U.S.A., David kopf). Scalp was incised and the bone of skull was exposed. Guide cannula 25G (Eicom Inc., Japan) was implanted 4.5 mm the above the left red nucleus and fixed with dental cement. After 2 - 3 days, injection cannula 27G (Eicom Inc., Japan) was inserted into the guide cannula without anesthetization. The tip of the cannula was introduced into the red nucleus below 4.5 mm of the guided cannula. Haloperidol (Sigma Inc., U.S.A.) 3 nmol was infused into the red nucleus from injection cannula using injection pump (Harvard Apparatus Inc., U.S.A.) during 72 seconds.

Rats were set into the observation cage made with transparent acrylic resin and recorded the front view image by video camera. Measurement of dystonia was performed by measuring the angle between a horizontal line and a line connecting both eyes by computer and analyzing as the torsional angle of the head. After measurement, ink was infused through the injection cannula and the brain slices were prepared. The infused position was confirmed to be the red nucleus by the slices. The torsional angle of the head as a result of haloperidol 3 nmol infusion was defined as control group and combined infusion of haloperidol 3 nmol and the present compound 0.03 nmol was defined as the compound A combined group. Result is shown as mean ± S.D. of the maximum torsional angle of 12 rats.

### 2. Result

Result is shown in Table 6.

**Table 6**

| Drug | torsional angle of head |
|---|---|
| Control | 24.1 ± 1.7 |
| Compound A combined group | 15.5 ± 1.3** |

| | |
|---|---|
| ** : p<0.01 | |

Result indicates that the present compound shows a strong inhibitory effect in the dystonia model. Consequently, the present compound was confirmed to have a marked suppressive action against abnormal movements characterized by slow involuntary movement and hypermyotonia as like dystonia.

### Example 5

### Effect on the firing rate in the red nucleus

The red nucleus belongs extrapyramidal system and have an important role for regulating motor function. Destruction of the red nucleus in rats was reported to enhance the catalepsy. (Kolasa. K. et al., Pharmacol. Biochem. Behav.,51, 29 - 35, 1995). As shown in Example 4 dystonia model, unilateral infusion of haloperidol into red nucleus of rats results to appear dystonia. As shown like this, the hypofunction of red nucleus have a close relation with the expression of abnormal movements accompanying disorders of extrapyramidal system. Namely, hypofunction of red nucleus is considered to lead to the abnormal movements accompanying disorder of extrapyramidal system. In this experiment, effect of the compound of the present invention on the haloperidol-induced decrease of firing rate in red nucleus was examined.

### 1. Method

Wistar rats, male (Japan Charles River Inc., 7- 9 weeks old) were anesthetized by intraperitoneal administration of urethane (Tokyo Kasei K.K., Japan) 1.2 g/kg. Right femoral vein was exposed and catheter for administration of drug (PE50, Beckton Deckinson Inc., U.S.A.) was inserted into the vein. The head of rats were mounted in stereotaxic apparatus (SR-6, Narishige Inc., Japan), and body temperature was kept at 37°C by heating pad. The skull was exposed and the position, of the skull above the red nucleus was drilled a smallhole, then dura and arachnoid were incised. Micro glass electrode (tip diameter about 1 µm) filled with 2M NaCl solution containing 2 % pontamine skyblue 6B (Tokyo Kasei K.K., Japan) was inserted into the depth of 6.5 - 7.5 mm in the red nucleus vertically from surface of brain guided by the manipulator (SM-15, Narishige Inc., Japan).

Electric signals from the glass electrode were amplified with a micro electrode amplifier (MEZ-8301, Nihon Koden Co., Japan), filtered band path of 3 kHz - 50 Hz and humfilter and monitored on a oscilloscope (VC-11, Nihon Koden Co., Japan). Spontaneous firing of neronal cells in red nucleus was observed as positive change over 0.2 mV, 9 - 30 times/sec. Number of firing/10 seconds (firing rate) was calculated using pulse counter (MET-1100, Nihon Koden Co., Japan) and recorded by computer (Maclab ver. 3.5. Macintosh) with time dependent manner.

After confirming stable firing of red nucleus, saline or 0.1 µg/kg of the compound of the present invention were administered through the catheter intravenously, and after 1 - 3 minutes, each 0.2 mg/kg of haloperidol was administered intravenously. Firing rate was continuously recorded before the administration of the compound of the present invention. Effect of the compound of the present invention was determined as follows. Firing rate of red nucleus is defined as 100 % before administration of the compound of the present invention or saline, and percentage of inhibition on the firing rate after administration of haloperidol was expressed. After completion of the experiment, the slices were prepared from extracted brain and position of the electrode was confirmed to be correct.

### 2. Results

Result of experiment is shown in Table 7.

**Table 7**

| Drug | % Inhibitor of firing rate |
|---|---|
| Saline + haloperidol | 60.1 % |
| Compound A (hydrochloride) + haloperidol | 24.7 %* |

| | |
|---|---|
| * P<0.05 | |

The red nucleus receives the input from cerebral cortex, superior colliculus, cerebellum and pallidum. The pallidum receives neuronal connection from nigrostriatal pathway. Also the output from red nucleus connected to olivary nucleus and spinal cord. Triangle circuit from red nucleus to olivary nucleus, from olivary nucleus to cerebellum, and from cerebellum to red nucleus, is constituted. Olivary nucleus and cerebellum are close relationship with motor function. Consequently, the red nucleus lies in the lower position as the center of the extrapyramidal system, and has important role to transmit the information from basal nuclei and cerebellum to olivary nucleus and peripheral nerve. Haloperidol, which induce abnormal movements, significantly suppressed the firing rate of red nucleus in rats. The compound of the present invention prevented the inhibitory action of haloperidol. Namely, effectiveness of the present compound on abnormal movements accompanying disorder of extrapyramidal system was proved by electrophysiological study.

As clearly understand from these experimental results hereinbefore, the compound A of the present invention or non-toxic salt thereof is obviously effective on the abnormal movements acompanying disorders of extrapyramidal system caused by various factors.

### Effect of the invention

The present invention provides preventives/remedies for abnormal movements accompanying disorders of extrapyramidal system, comprising (1S)-3-[2-(2-adamantyl)ethyl]-1,8,8-trimethyl-3-azabicyclo [3.2.1]-octane or a pharmacologically acceptable acid-addition salt thereof as an active ingredient.

## Claims

1. Preventives/remedies for abnormal movements accompanying disorders in the extrapyramidal system comprising (1S)-3-[2-(2-adamantyl) ethyl]-1,8,8-trimethyl-3-azabicyclo [3.2.1]-octane or a pharmacologically acceptable acid-addition salt thereof as an active ingredient.

2. The preventives/remedies according to claim 1 wherein the abnormal movements accompanying disorders in the extrapyramidal system are the abnormal movements in more than any one of the abnormal movement with major symptom of tremor, muscle rigidity or hypokinesia; the abnormal movement with major symptom of rapid involuntary movement and muscle hypotonia; and the abnormal movement with major symptom of slow involuntary movement and hypermyotonia.

3. The preventives/remedies according to claim 1 or claim 2 wherein the abnormal movements accompanying disorders in the extrapyramidal system are the abnormal movements selected from the group consisting of tremor, muscle rigidity, hypokinesia, chorea, ballismus, dyskinesia, akathisia, dystonia and athetosis.

4. The preventives or remedies according to claims 1 to 3 wherein the said abnormal movements accompanying disorders in the extrapyramidal system are side effect of drug abnormal movements.

5. Preventives/remedies comprising (1S)-3-[2-(2-adamantyl) ethyl]-1,8,8-trimethyl-3-azabicyclo [3.2.1]-octane or a pharmacologically acceptable acid-addition salt thereof as the active ingredient, for disease selected from the group consisting of Parkinson's disease, striate-nigral degeneration, olivopontocerebellar atrophy, Shy-Drager syndrome, progressive supranuclear palsy, essential tremor, Huntington's chorea, senile chorea, denate-rubro-pallido-luysian atrophy, benign familial chorea-acanthocytosis dystonia musculorum deformans, Meige's syndrome and Segawa disease.
